# EUROPEAN PATENT SPECIFICATION

(11) **EP 0 826 664 B1**
(45) Date of publication and mention of the grant of the patent: **20.06.2001**
(21) Application number: 97905454.1
(22) Date of filing: 06.03.1997
(51) Int. Cl.: C07C 233/47, C07C 231/02, C07C 231/24

(54) **PROCESS FOR THE PREPARATION OF N OMEGA-TRIFLUOROACETYLATED BASIC AMINO ACIDS**
VERFAHREN ZUR HERSTELLUNG VON N OMEGA-TRIFLUORACETYLIERTEN BASISCHEN AMINOSÄUREN
PROCEDE POUR PREPARER DES AMINOACIDES BASIQUES N OMEGA-TRIFLUOROACETYLES

(30) Priority: 08.03.1996 JP 5184696
(43) Date of publication of application: 04.03.1998
(60) Divisional application: 00120296.9
(73) Proprietor: KANEKA CORPORATION, Osaka-shi, Osaka 530-8288 (JP)
(72) Inventor: UEDA, Yasuyoshi, Hyogo 671-12 (JP); MANABE, Hajime, Hyogo 676 (JP); MITSUDA, Masaru, Hyogo 676 (JP)
(74) Representative: VOSSIUS & PARTNER
(86) International application number: JP9700690
(87) International publication number: WO9732841

(56) References cited:
- EP-A- 0 239 063
- EP-A- 0 279 716
- JP-A- 62 226 952

## Description

The present invention relates to a process for preparation of N^{ω}-trifluoroacetyl-basic amino acid by selectively introducing a trifluoroacetyl group as a protecting group into only side chain amino group (N^{ω}) of a basic amino acid. More particularly, it relates to a process for preparing high quality of N^{ω}-trifluoroacetyl derivatives of ornithine, lysine and homolysine, which derivatives are useful as a synthetic raw material for physiologically active peptides, in a high yield with good economy on a commercial scale.

### BACKGROUND ART

As a process for preparation of N^{ω}-trifluoroacetyl-basic amino acid by selectively introducing a trifluoroacetyl group as a protecting group into only side chain amino group (N^{ω}) of a basic amino acid, particularly, a process for preparing N^{ω}-trifluoroacetyl derivatives of lysine and ornithine, which derivatives are useful as a synthetic raw material for physiologically active peptides, there has been hitherto known a process which utilizes trans-trifluoroacetylation of trifluoroacetic ester shown by the following reaction scheme, as described in JP-A 62-226952.

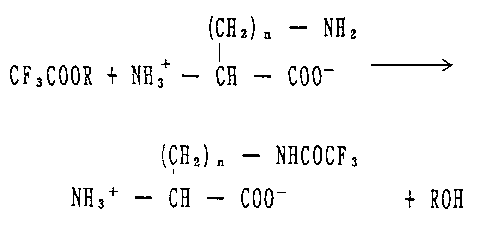

In the above reaction scheme, R is an alkyl group, an aralkyl group or an aryl group, or substituted groups thereof, and n = 1 to 6.

As this process utilizing trans-trifluoroacetylation, the above-mentioned JP-A 62-226952 describes a so-called reactive crystallization process which comprises reacting a basic amino acid and a trifluoroacetic ester in an aqueous solution while vigorously stirring and collecting N^{ω}-trifluoroacetyl-basic amino acid which is separated out with the progress of the reaction.

In the aforementioned process utilizing trans-trifluoroacetylation, N^{ω}-trifluoroacetyl-basic amino acid which is formed with the progress of the reaction is gradually precipitated in the form of slurry (whip or cake). For this reason, there is a problem that the stirring efficacy during the reaction is reduced and sufficient mixing cannot be performed, which leads to a reduced yield.

In addition, there are problems that since a product is precipitated with the progress of the reaction and the reaction mixture becomes whip-like or cake-like, special and expensive reaction facilities are required in order to stir vigorously to obtain the sufficient stirring efficacy and that the reaction concentration cannot be raised.

Further, as a method for collecting crystals of N^{ω}-trifluoroacetyl-basic amino acid, the above-mentioned JP-A 62-226952 describes a collecting method by the reactive crystallization in which after the reaction, the precipitated crystals of N^{ω}-trifluoroacetyl-basic amino acid are subsequently collected. In this case, there is a problem on the operability and the productivity that since fluidity of the slurry is not good, discharge from a reaction vessel is very difficult, the filtration property (crystal separating property), the washing property and the dehydrating property are greatly reduced, the crystal separation takes a long time, and the drying of wet cake (wet crystals) takes a long time due to shrinkage and dumpling formation. Further, there is also a problem on the productivity, the yield and the quality that since low quality N^{ω}-trifluoroacetyl-basic amino acid containing impurities such as salts produced during reaction to crystallization and unreacted basic amino acid is obtained, it is required to perform recrystallization in order to attain the high quality.

Although it can be envisaged that a part of the aforementioned problems is overcome by reducing the reaction concentration and the crystallization concentration (i.e., formation of whip and cake is avoided to improve the stirring efficacy and the fluidity of the reaction mixture), it is required to greatly reduce the reaction concentration in order to make commercial scale production possible and, therefore, which leads to another problems such as remarkable reduction in the yield and productivity, and the necessity of large volume of the reaction facility.

In Example 1 of the above-mentioned JP-A 62-226952, there is disclosed a high concentration reaction wherein N^{ω}-trifluoroacetyl-L-lysine is obtained by reacting L-lysine with 1.5-fold moles of ethyl trifluoroacetate. However the yield is 58 mol% based on L-lysine and 39 mol% based on ethyl trifluoroacetate and, and when the recovery of the second crop by concentration of the filtrate and washing solution, the yield is 77 mol% based on L-lysine and 52 mol% based on ethyl trifluoroacetate. Upon this, since the resultant N^{ω}-trifluoroacetyl-L-lysine has a deteriorated crystal property, the productivity is inferior and there arises a problem also on the quality that the quality is deteriorated due to contamination of inorganic salts and the like. When recrystallization is performed in order to obtain high quality N^{ω}-trifluoroacetyl-L-lysine, the total yield is 55 mol% based on L-lysine and only 36 mol% based on ethyl trifluoroacetate. In the process disclosed in the aforementioned JP-A 62-226952, in order to raise the yield of N^{ω}-trifluoroacetyl-L-lysine, it is required to use a largely excess amount of expensive trifluoroacetic ester and, in this case, there is also a problem that the treatment of fluorine-containing wastewater has an elevated load.

The various problems described above become a serious matter in dealing with a large amount of N^{ω}-trifluoroacetyl-basic amino acid, in particular, as a production process on a commercial scale.

The object of the present invention is to provide a process for preparation of N^{ω}-trifluoroacetyl-basic amino acid by reacting a basic amino acid with a trifluoroacetic ester in an aqueous liquid, by which the above problems can be overcome and high quality N^{ω}-trifluoroacetyl-basic amino acid is prepared in a high yield with good economy on a commercial scale.

### DISCLOSURE OF THE INVENTION

In view of the above-mentioned circumstances, the present inventors extensively have studied in order to solve the above problems and, as a result, found that, in a process for preparation of N^{ω}-trifluoroacetyl-basic amino acid from a basic amino acid and a trifluoroacetic ester, when the reaction is performed by adding a trifluoroacetic ester to an aqueous liquid containing a basic amino acid and a basic pH adjusting agent under a specified condition, the slurrying (formation of whip and cake) is suppressed even in a high reaction concentration and a reaction mixture of good liquid condition can be obtained and, therefore, a load in terms of stirring efficacy can be considerably reduced and, additionally, a high reaction yield can be attained and, further, by neutralizing or acidifying the resultant mixture and growing good crystals while warming, formation of whip and cake can be suppressed to give a slurry having good fluidity and, at the same time, high purity N^{ω}-trifluoroacetyl-basic amino acid having excellent crystal property excellent in the filtration property (crystal separating property), the washing property, the dehydrating property and the drying property can be prepared in a high yield, which resulted in completion of the present invention.

That is, the present invention provides a process for preparation of N^{ω}-trifluoacetyl-basic amino acid as defined in the claims.

Furthermore, the present invention provides a process for crystallizing and collecting N^{ω}-trifluoroacetyl-basic amino acid.

In the present invention, N^{ω}-trifluoroacetyl-basic amino acid is prepared by a reaction of a trifluoroacetic ester and a basic amino acid. Upon this reaction, a trifluoroacetic ester is added to a basic aqueous liquid containing a basic amino acid and a basic pH adjusting agent.

As the trifluoroacetic ester, there can be used various esters including, for example, alkyl esters wherein examples of the alkyl group are those having 1 to 8 carbon atoms such as methyl, ethyl, n-propyl, isopropyl, n-butyl, isobutyl and sec-butyl, aralkyl esters wherein examples of the aralkyl group are those having 7 to 12 carbon atoms such as benzyl and phenethyl, and aryl esters or substituted aryl esters wherein examples of the aryl or substituted aryl group are those having 6 to 10 carbon atoms such as phenyl and p-nitrophenyl. In view of the operability and the like, the use of alkyl esters whose alkyl group has 2 to 4 carbon atoms is preferable and ethyl trifluoroacetate is particularly preferably used. These trifluoroacetic esters may be used either alone or in a combination of two or more species thereof.

The basic amino acid is represented by the following general formula (I): 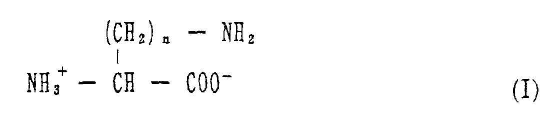 wherein n represents an integer of 1 to 6. Preferable representative examples are ornithine (n = 3), lysine (n = 4) and homolysine (n = 5) and, inter alia, lysine is preferably used. Although these may be used in the form of L compound, D compound or DL mixture, L or D optically active compound is preferably used from the viewpoint of the industrial applicability. In addition, from the viewpoint of easy handling, the contribution to salting-out effect at crystallization and the like, they are preferably used as a salt of a basic amino acid (particularly preferably a salt with a strong acid) including, for example, hydrochloride such as L-lysine hydrochloride.

Examples of the pH adjusting agent are inorganic bases. Examples of the inorganic base are alkali metal hydroxides and alkali metal carbonates. Examples of the alkali metal hydroxide are sodium hydroxide, potassium hydroxide and lithium hydroxide. Examples of the alkali metal carbonate are sodium carbonate, potassium carbonate and lithium carbonate. From an overall viewpoint such as easy handling, an increase in the amount of crystals of a desired product to be crystallized due to salting-out effect of a salt which is formed at the crystallization step by neutralization, and an increase in the reaction concentration, sodium hydroxide, potassium hydroxide and lithium hydroxide are preferable and sodium hydroxide is particularly preferable. From the viewpoint of the operability and the like, the inorganic bases are preferably used in the form of an aqueous solution such as an about 2 to 20 N aqueous solution of sodium hydroxide (an about 9 to 10 N aqueous solution of sodium hydroxide is particularly preferably used from the viewpoint of easy handling, and an increase in yield and reaction concentration). The basic pH adjusting agents may be used either alone or in a combination of two or more species thereof. In addition, the basic pH adjusting agents are not limited to the aforementioned ones and, for example, organic bases such as triethylamine and pyridine can be used as required.

The amount of the basic pH adjusting agent to be used is an amount required for adjusting the pH of the basic aqueous liquid to a value within the range of 10.6 to 11.4.

Examples of the solvents for the basic aqueous liquid are water and a mixture of water and an organic solvent. The use of only water as the solvent is preferable since a high concentration reaction can be performed wherein the solubility of a raw material such as ornithine, lysine or homolysine and that of a product, which is a salt of N^{ω}-trifluoroacetyl-basic amino acid such as N^{ω}-trifluoroacetyl derivative of ornithine, lysine or homolysine, are maintained high; the amount of crystals of a desired product to be crystallized due to salting-out effect in a salt solution (for example, sodium chloride) in the crystallization can be increased; high concentration crystallization can be performed; post-treatment including wastewater treatment can be easily performed without using a harmful organic solvent; and the cost is low.

As required, a mixture of water and an organic solvent may be used. When a mixture of water and an organic solvent is used as a solvent, a system may be one where an organic solvent miscible with water and water are uniformly mixed, or an ununiform two-phase system comprising an organic solvent immiscible with water and water.

Examples of the organic solvent are ethers such as diethyl ether and tetrahydrofuran, halogenated hydrocarbons such as methylene chloride and dichloroethane, hydrocarbons such as toluene, methylcyclohexane and n-hexane, alcohols such as methanol, ethanol, propanol and butanol, acetate esters such as methyl acetate, ethyl acetate, propyl acetate and butyl acetate, ketones such as acetone and methyl ethyl ketone, aprotic polar solvents such as dimethylformamide, dimethyl sulfoxide and N-methylpyrrolidone. However, the organic solvents are not limited thereto. In general, the organic solvents are preferably water-miscible ones from the viewpoint of increase in reaction rate and the like.

The basic aqueous liquid refers to an aqueous liquid which is prepared by adding a basic amino acid and a basic pH adjusting agent to water or a mixture of water and an organic solvent as a solvent and which is made basic with the basic pH adjusting agent added together with the basic amino acid. A base is not necessarily required to be added thereto in order to make an aqueous liquid basic. However, in order to make pH adjustment easy during the reaction, a basic aqueous solution having a buffer capacity which is obtained by adding disodium hydrogen phosphate, hydrochloric acid, boric acid or the like may be used. In addition, the basic aqueous liquid may be incorporated with a surfactant, a phase transfer catalyst or the like as required.

In the present invention, the reaction between a trifluoroacetic ester and a basic amino acid is performed while maintaining the pH of the reaction mixture at a value within the range of 10.6 to 11.4, preferably within the range of 10.8 to 11.2 from the viewpoint of an improvement on the yield and the quality. Performance of the reaction in a suitable pH range is an important factor in the present invention. When pH in the reaction becomes lower than 10.6, a product is separated out and the reaction mixture is gradually changed into a slurry (whip-like or cake-like) and, thereby, the stirring efficacy in the reaction is reduced and sufficient stirring cannot be performed, which leads to such a tendency that the reaction yield is reduced. On the other hand, when pH in the reaction becomes higher than 11.4, separating-out of a product can be suppressed but alkali-hydrolysis of trifluoroacetic ester as a raw material and a product (detrifluoroacetylation) are prone to progress, which leads to such a tendency that the reaction yield is reduced. As the results of study by the present inventors, it has been found that the pH value to be selected from an overall viewpoint of the reaction yield, the reaction selectivity at ω / α -positions, the stability of a product, the liquid condition of a reaction mixture and a reduction in a volume of the reaction mixture is about pH 11.

Hitherto, isoelectric points (pI) of both lysine and ornithine which are representative basic amino acids have been known to be 9.7 (see "Revised 4th Edition, Kagakubinran Kisohen I". edited by the Chemical Society of Japan, published by Maruzen, pages 431-434 (1993)). In order to enhance the selectivity at ω / α positions by selectively protonating an amino acid at α -position and maintaining an amino acid at ω-position in an undissociated state, around pH 9.7 is considered to be a preferable pH region in both cases. Although JP-A 62-226952 describes that pH 8 to 11 is preferable for improving the selectivity at *ω l α* positions and J. Org. Chem., Volume 53, pages 836-844 (1988) describes that a pH region higher than pH 11 is preferable in order to enhance the selectivity at ω / α positions, preferable pH is not reported from the viewpoint of the reaction yield, the stability of a product, the liquid condition of a reaction mixture and a reduction in a volume amount of a reaction mixture and in increasing of the reaction concentration.

A molar ratio of a trifluoroacetic ester relative to a basic amino acid is usually 0.5 to 2. However, under the conditions according to the present invention, a molar ratio approximate to 1 is most preferably used from the viewpoint of the quality of the resulting product, the load in wastewater treatment and economy (usually 0.7 to 1.2, preferably 0.8 to 1.1 from the viewpoint of the quality and economy, particularly preferably 0.9 to 1.0). As a result, the yield relative to trifluoroacetic ester is improved.

It is preferable that the total amount of a basic amino acid is added at the start of the reaction from the viewpoint that a high yield can be insured and the operability can be simplified.

The trifluoroacetic ester is preferably added alone as it is from the viewpoint of easy wastewater treatment and an increase in a reaction concentration. As required, for example, the trifluoroacetic ester may be added in a state wherein it is dissolved in a liquid having no adverse effect on the reaction which is appropriately selected from the aforementioned organic solvents (for example, tetrahydrofuran and alcohols). It is not preferable that trifluoroacetic ester or a solution thereof is reacted for a longer period of time after the addition in order to attain a high yield on a commercial scale. Instead, it is preferable that the reaction is performed while adding trifluoroacetic ester in succession. Although it depends upon a reaction temperature or the like, it is preferable from the viewpoint of improving the yield and quality that the reaction is performed by successively adding trifluorocetic ester such that the total amount thereof is added usually in about 1/4 to 8 hours, preferably about 1/3 to 6 hours, particularly about 1/2 to 3 hours (about 1 hour being most preferable). Examples of a preferable method for successive addition include a method of addition at a constant rate and a method of portionwise addition. In particular, a method of addition at a constant rate is preferable from the viewpoint of improvements on yield, quality and operability.

The reaction temperature is usually not higher than about 20°C, preferably not higher than about 10°C from the viewpoint of improvements on yield and quality, particularly preferably not higher than about 5°C. A lower limit is a temperature at which a reaction mixture is not frozen. When the reaction temperature is too high, hydrolysis of a raw material trifluoroacetic ester and a product (detrifluoroacetylation) is prone to occur, which results in a reduction in yield.

Upon reaction, it is preferable that stirring and mixing are performed so that reactants are suitably dispersed. However, since a reaction mixture of a good liquid condition is obtained by the reaction conditions according to the present invention, such a strong stirring is not required that a slurry (whip-like or cake-like) reaction mixture is stirred and mixed. Stirring is performed at an agitation power of about 1/10 kW/m³ or more, preferably about 1/3 kW/m³ or more from the viewpoint of improvements on yield and quality, particularly preferably about 1/2 kW/m³ or more. An upper limit of about 5 kW/m³ is preferable from the practicability. Usually, the agitation power can be preferably selected from a range of about 1/3 to 3kW/m³.

As regards a charging concentration, a total weight of a basic amino acid relative to a volume of a charged solvent is about 5 to 40 % (w/v), usually about 10 % (w/v) or more from a viewpoint of the productivity, preferably about 15 % (w/v) or more. For example, in the case of preparation of N^{ω}-trifluoroacetyl-L-lysine, this can afford a high concentration reaction mixture having a product concentration of about 10 % (w/v) or more and having better fluidity. Obtaining of such a high concentration reaction mixture having better fluidity contributes to not only an improvement on the productivity such as an improvement on the operability and a reduction in a volume of a reaction mixture, but also an increase in the amount of a product to be crystallized.

In addition, the reaction yield (formation rate) in preparation of the aforementioned N^{ω}-trifluoroacetyl-basic amino acid is expected to be about 75 mol% or more, preferably about 80 mol% or more.

The reaction in the present invention can be conducted also in an inert atmosphere (for example, nitrogen or argon).

According to the present invention, an aqueous reaction mixture containing N^{ω}-trifluoroacetyl-basic amino acid at a high concentration (preferably about 0.5 mol/1 or more) and in a liquid condition suitable for operability and the subsequent crystallization is obtained at a high reaction formation rate and a high ω selectivity without using a largely excess amount of trifluoroacetic ester relative to basic amino acid (see Example 3 in JP-B 6-15514).

Next, the process for crystallization and collection of N^{ω}-trifluoroacetyl-basic amino acid from the aqueous liquid after the reaction will be explained.

The aqueous liquid after the reaction is neutralized or acidified. The aqueous solution is neutralized or acidified to a pH value of about 8 or lower, preferably a pH value of about 7 or lower, where N^{ω}-trifluoroacetyl-basic amino acid is stable (that is, detrifluoroacetylation can be suppressed).

As regards the acid used for acidification or neutralization, strong acids are preferable from the viewpoint of the practicability. Usually, mineral acids such as hydrochloric acid and sulfuric acid are preferable. Particularly, hydrochloric acid is preferably used (concentrated hydrochloric acid is most preferable). However, acids to be used are not limited thereto. These may be used either alone or in a combination of two or more species thereof. The amount of an acid to be used is an amount required for neutralizing (unnecessary) basic components which are not preferable upon collection of N^{ω} -trifluoroacetyl-basic amino acid crystals or an excess amount.

Then, crystals are grown while maintaining the temperature at 40°C or higher and adjusting the pH to around the isoelectric point of N^{ω}-trifluoroacetyl-basic amino acid, that is, a pH value within the range of the isoelectric point ± 3 as described below.

In order to obtain high purity crystals having good crystal property and large size, crystals are grown while maintaining the temperature at not lower than 40°C, usually, at 40° to 100°C, preferably 50° to 90°C from the viewpoint of the operability and obtaining of better crystals, particularly preferably 60° to 80°C.

A slurry having better fluidity can be obtained by slowly growing crystals for, usually, about 1/3 hour or longer, preferably 1 to 3 hours or longer.

During this operation, the growth of crystals is performed while maintaining the pH at around the isoelectric point when the pH of the aqueous liquid is around the isoelectric point or while slowly adjusting the pH to around the isoelectric point when the pH of the aqueous liquid is on an acidic side. The pH value varies more or less depending upon the isoelectric points of N^{ω}-trifluoroacetyl-basic amino acids and is usually around pH 4 to 7, preferably around pH 5 to 6 from the viewpoint of an increase in a crystallized amount. A pH value of about 5.5 ± 0.5 is most preferable. At this pH range, a crystallized amount is maximum.

As the base used for adjusting the pH, inorganic bases are preferable. Usually, alkali metal hydroxides such as sodium hydroxide, potassium hydroxide and lithium hydroxide, alkali metal carbonates such as sodium carbonate, potassium carbonate and lithium carbonate, alkali metal hydrogencarbonates such as sodium hydrogencarbonate, potassium hydrogencarbonate and lithium hydrogencarbonate, ammonium hydroxide, and the like are used. Among them, sodium hydroxide, potassium hydroxide and lithium hydroxide are preferable from the viewpoint of the practicability and sodium hydroxide is particularly preferable. The use of the aforementioned bases as an aqueous solution such as about 2 to 20 N aqueous solution of sodium hydroxide (about 9 to 10N aqueous solution of sodium hydroxide is particularly preferably used) is preferable from the viewpoint of the operability. Bases to be used are not limited thereto. These bases may be used either alone or in a combination of two or more species thereof.

The use of preferable acid components and base components at the reaction to crystallization steps is important in order to produce inorganic salts which can be easily disposed and, at the same time, increase a yield of N^{ω}-trifluoroacetyl-basic amino acid due to salting-out effect of the produced salts. Inorganic salts, particularly sodium chloride is effective in salting-out.

Alternatively, upon neutralization or acidification, ion exchange resins (Dowex 50™ (H⁺ type) manufactured by Dow Chemical Co., and the like) can be used. N^{ω}-trifluoroacetyl-basic amino acid can be crystallized by contacting a reaction mixture containing N^{ω}-trifluoroacetyl-basic amino acid with an ion exchange resin and concentrating the finally obtained solution according to a usual method.

The crystal growth is preferably performed by mildly stirring from the viewpoint of the crystal property and the slurry condition, particularly preferably by stirring at a minimum level at which the slurry flows. Usually, stirring is performed at an agitation power of about 0.01 to 0.5 kW/m³, preferably about 0.05 to 0.2 kW/m³. The aforementioned temperature at the crystal growth affords a slurry having good fluidity which is suitable for stirring. This can considerably suppress formation of floating crystals and a tendency of the conversion of the slurry into whip or cake and can aid in obtaining preferable large size and high quality crystals having settling property and a slurry having good fluidity.

Upon collection of N^{ω}-trifluoroacetyl-basic amino acid crystals, the slurry can be finally cooled to further increase a crystallized amount. Upon this, cooling to about 20°C or lower, usually to about 0° to 20°C (particularly around 10°C ) is preferable from the viewpoint of the operability and an increase in a crystallized amount.

In addition, a crystallization yield upon the aforementioned crystallization of N^{ω}-trifluoroacetyl-basic amino acid is expected to be about 75 mol% or more, preferably about 80 mol% or more based on N^{ω}-trifluoroacetyl-basic amino acid being present.

The crystallizing method in the present invention can be performed also in an inert atmosphere (for example, nitrogen or argon).

By the crystallizing method in the present invention, a slurry having good fluidity even at a crystal concentration (slurry concentration) of 10 % (w/v) or higher relative to the crystallization solution can be obtained. Obtaining of such high concentration slurry having good fluidity contributes to not only productivity such as an improvement on the operability and a reduction in a volume of a reaction mixture but also an increase in a crystallized amount.

In addition, by the crystallizing method in the present invention, high quality N^{ω}-trifluoroacetyl-basic amino acid can be obtained simply and effectively without recrystallization.

Preferable fundamental embodiment of the present invention will be described by way of preparation of N^{ω}-trifluoroacetyl-L-lysine but the present invention is not limited thereto.

L-lysine monohydrochloride is added to about 3 to 4-fold weight of water, and the pH is adjusted to about 11 using an about 10 N aqueous solution of sodium hydroxide. The reaction is performed at an agitation power of about 1 kW/m³ while adding ethyl trifluoroacetate, the amount of which is about 0.9-fold mole based on L-lysine monohydrochloride, to the mixture at approximately constant rate over 1 to 2 hours. During the reaction, the reaction temperature is maintained at about 0° to 6°C and the pH of the reaction mixture is maintained at about 11 (usually in a range of 10.8 to 11.2) by adding an about 10 N aqueous solution of sodium hydroxide thereto. After the addition of ethyl trifluoroacetate, the reaction mixture is acidified or neutralized.

The reaction mixture (about 0° to 6°C ) is added to about 2-fold mole of a concentrated hydrochloric acid (about 8° to 35°C ) relative to the amount of ethyl trifluoroacetate used, which is then warmed to about 60° to 80°C. Further, about 10 N aqueous solution of sodium hydroxide is added thereto at an approximately constant rate over about 1 hour to adjust the pH to about 5.5 to grow and crystallize N^{ω}-trifluoroacetyl-L-lysine crystals, followed by cooling to about 15°C or lower to complete crystallization. The agitation power upon crystallization is about 0.1 kW/m³.

Alternatively, to a concentrated hydrochloric acid (about 60° to 80°C ) in an amount which makes the pH of the reaction mixture about 5.5 is added the reaction mixture (about 0°C ) at an approximately constant rate over 1 hour to adjust the pH to about 5.5 to grow and crystallize N^{ω}-trifluoroacetyl-L-lysine crystals. As required, the pH is finely adjusted with a concentrated hydrochloric acid or an about 10 N aqueous solution of sodium hydroxide, followed by cooling to about 15°C or lower to complete crystallization. The agitation power upon crystallization is about 0.1 kW/m³.

The sodium chloride concentration in the resulting slurry is about 10 % (w/w) or higher, which results in a higher yield of N^{ω}-trifluoroacetyl-L-lysine crystallized.

In addition, upon crystallization of N^{ω}-trifluoroacetyl-basic amino acid, an inorganic salt having salting-out effect (sodium chloride having high salting-out effect is most preferable) may be additionally present as required, or N^{ω}-trifluoroacetyl-basic amino acid contained in the filtrate and/or the washing solution may be recovered and recycled. This can minimize the loss of a product into a wastewater.

From the thus obtained crystallization solution (slurry) of N^{ω}-trifluoroacetyl-basic amino acid crystals can be collected the crystals using a conventional crystal separating method such as filtration under pressure, filtration under reduced pressure or centrifugal separation (centrifugal separation is most preferable from the viewpoint of productivity). Further, the separated cake can be washed with water and/or an organic solvent. Alternatively, the separated cake can be washed with cooled water in order to suppress the loss of a product into a washing solution. Further, in order to facilitate drying, the separated cake can be washed with an organic solvent. As the aforementioned organic solvent, absolute or water-containing alcohols (water content of, usually, about 5 to 50 % (w/w), particularly about 5 to 10 % (w/w) is preferably used from the viewpoint of the practicability) can be used. As the aforementioned alcohol, absolute or water-containing alcohols having 1 to 3 carbon atoms, particularly ethanol can be preferably used. It has been found that washing and removal of the filtrate (mother liquor) contained in wet crystals with the aforementioned alcohol minimizes the loss of a product and facilitates drying. Since large size N^{ω}-trifluoroacetyl-basic amino acid crystals having good crystal property can be obtained according to the crystallizing method in the present invention, the aforementioned washing with alcohols is sufficiently done and salts in the filtrate contained in the cake may be effectively washed off.

The resulting N^{ω}-trifluoroacetyl-basic amino acid crystals can be finally dried under normal pressure or reduced pressure. Further, the crystals can be pulverized and granulated as required.

N^{ω}-trifluoroacetyl-basic amino acid prepared according to the present invention can be converted into physiologically active peptides according to the method, for example, described in JP-A 61-36297.

### BEST MODE FOR CARRYING OUT THE INVENTION

The following Examples further describe the present invention in detail but the present invention is not limited thereto.

### EXAMPLE 1

To 135 ml of water was added 35.05 g (192 mmol) of L-lysine monohydrochloride and the resultant was adjusted to pH 11.0 with a 9.5N aqueous sodium hydroxide solution (217 mmol). The solution was cooled to an inner temperature of 0° to 3°C, and 24.35 g (171 mmol) of ethyl trifluoroacetate was slowly added dropwise thereto under stirring over 1 hour. During the addition, the reaction mixture was maintained at pH 10.8 to 11.2 while adding a 9.5N aqueous sodium hydroxide solution (130 mmol), and the inner temperature and the agitation power were maintained at 0° to 3°C and 1.5 kW/m³, respectively. After the addition of ethyl trifluoroacetate was completed, stirring was continued for 20 minutes under the same conditions. The reaction mixture (pH 11.1) having good liquid condition without precipitation of the product was obtained. The reaction mixture and 4.0 g (17 mmol) of the second crop obtained in a separate experiment were added together to 35 g of a concentrated hydrochloric acid to dissolve them. The pH after the addition was 1.0 or lower. Rinsing with 6 ml of water was performed. The mixture was warmed to 70°C and an about 9.5 N aqueous sodium hydroxide solution (164 mmol) was added thereto at an approximately constant rate over 1 hour to adjust the pH to 5.5 (stirring was conducted at a lowest level (0.1 to 0.2 kW/m³) at which the slurry could be agitated. Crystal precipitation initiated at around pH 2). The mixture was cooled to an inner temperature of 15°C or lower and crystals were centrifuged and washed with 25 g of 93 % ethanol. The separating property was very good. The crystals were vacuum-dried under warming at the highest temperature of 70°C. The yield of N^{ω}-trifluoroacetyl-L-lysine was 31.4 g (130 mmol) and the purity was 99% (w/w) or higher (N^{α}-isomer was not detected and sodium chloride content was 0.1% (w/w) or lower). 7.3 g (30 mmol) of N^{ω}-trifluoroacetyl-L-lysine was contained in the filtrate and washing solution. The mixture of the filtrate and washing solution was concentrated to 1/2 weight under reduced pressure at an inner temperature of about 50° to 80°C. After the concentration, the mixture was cooled to 30°C or lower to separate the crystals which were washed with 15 g of water. The yield of the second crop of N^{ω}-trifluoroacetyl-L-lysine was 4.0 g (17 mmol). The reaction yield (formation rate) was 84 mol%.

### EXAMPLE 2

To 700 ml of water was added 183 g (1.00 mol) of L-lysine monohydrochloride and the resultant was adjusted to pH 11.0 with a 9 N aqueous sodium hydroxide solution (1.2 mol). The solution was cooled to an inner temperature of 4°C, and 142 g (1.0 mol) of ethyl trifluoroacetate was slowly added dropwise thereto under stirring over 30 minutes. During the addition, the reaction mixture was maintained at pH 10.6 to 11.4 while adding a 9N aqueous sodium hydroxide solution (0.7 mol), and the inner temperature and the agitation power were maintained at 4°C and 0.5 to 1 kW/m³, respectively. After completion of the addition of ethyl trifluoroacetate, stirring was continued for 15 minutes under the same conditions. The reaction mixture (pH 11.1) having good liquid condition without: precipitation of the product was obtained. The cooled reaction mixture was added to 100 g of a concentrated hydrochloric acid warmed to 70°C over 1 hour. The addition was performed rapidly at the initial stage (particularly to around pH 1.3) and then gradually slowed. The pH after the addition was about 6. An extremely small amount of a 9N aqueous sodium hydroxide solution was added thereto to finely adjust the pH of the reaction mixture to 5.8. A little over 150 g of sodium chloride was added, followed by cooling to an inner temperature of 7°C over 1 hour. The agitation power upon crystallization was maintained at 0.1 kW/m³ (around minimum rate at which the slurry could be agitated). The crystals were filtered from the resultant slurry having good condition under reduced pressure using a nutsche having a diameter of 150 mm and washed with 200 ml of 93 % ethanol. The separating property was very good. The resultant crystals were dried at 60°C overnight to obtain N^{ω} -trifluoroacetyl-L-lysine crystals. The amount obtained was 165 g (total yield (indicated by reaction yield (formation rate) × crystallization yield, hereinafter the same): 68 mol%).

### EXAMPLE 3

To 80 ml of water was added 20.0 g (110 mmol) of L-lysine monohydrochloride and the resultant was adjusted to pH 11.2 to 11.3 with a 9.5 N aqueous sodium hydroxide solution. The mixture was cooled to an inner temperature of 0°C, and 15.6 g (110 mmol) of ethyl trifluoroacetate was slowly added dropwise thereto under stirring over 30 minutes. During the addition, the reaction mixture was maintained at pH 11.2 to 11.3 while adding a 9N aqueous sodium hydroxide solution dropwise thereto and, at the same time, the inner temperature and the agitation power were maintained at 0°C and 1.5 kW/m³, respectively. 30 minutes after completion of the addition of ethyl trifluoroacetate, the resultant reaction mixture having good liquid condition was added to 22 g of a concentrated hydrochloric acid all at once under room temperature. The pH after the addition was 1.0 or lower. The reaction mixture was warmed to 65°C, and a 9.5 N aqueous sodium hydroxide solution was added thereto at an approximately constant rate over 30 minutes under an agitation power of 0.2 to 0.5 kW/m³, to adjust the pH to 5.8. The reaction mixture was cooled to an inner temperature of 5°C, and the crystals were centrifuged and washed with 19 ml of 93 % ethanol. The separating property was very good. The amount of N^{ω}-trifluoroacetyl-L-lysine obtained was 16.5 g (68 mmol) (yield 62 mol%) and the purity was 99 % (w/w) or higher (N^{α}-isomer was not detected and sodium chloride content was 0.1 % (w/w) or lower). 4.5 g (19 mmol)(yield 17 mol%) of N^{ω}-trifluoroacetyl-L-lysine was contained in the filtrate and washing solution. The reaction yield (formation rate) was 79 mol%.

### EXAMPLE 4

The same procedures as in Example 3 were conducted using the mixture of the filtrate and the washing solution obtained in Example 3 in place of the water charged for the reaction. The amount of a concentrated hydrochloric acid used was 28 g. The reaction and crystallization could be performed in good liquid condition, and the amount of N^{ω}-trifluoroacetyl-L-lysine obtained was 19.6 g (81 mmol). 3.4 g (14 mmol) of N^{ω}-trifluoroacetyl-L-lysine was contained in the filtrate and washing solution.

### EXAMPLE 5

To 80 ml water was added 20.0 g (110 mmol) of L-lysine monohydrochloride and the resultant was adjusted to pH 10.9 to 11.1 with a 9.5 N aqueous sodium hydroxide solution. The mixture was cooled to an inner temperature of 11°C and 15.6 g (110 mmol) of ethyl trifluoroacetate was slowly added dropwise thereto under stirring over 20 minutes. During the addition, the reaction mixture was maintained at pH of 10.9 to 11.2 while adding a 9N aqueous sodium hydroxide solution dropwise thereto and, at the same time, the inner temperature and the agitation power were maintained at 9° to 14°C and 0.5 kW/m³, respectively. Ten minutes after completion of the addition of ethyl trifluoroacetate, the resultant reaction mixture having good liquid condition was added all at once to 22 g of a concentrated hydrochloric acid at room temperature. The pH after the addition was 1.0 or lower. The reaction mixture was warmed to 65°C, and a 9.5N aqueous sodium hydroxide solution was added thereto at an approximately constant rate under an agitation power of 0.2 kW/m³ over 30 minutes to adjust the pH to 5.5. The mixture was cooled to an inner temperature of 5°C and maintained at that temperature overnight. The crystals were centrifuged and washed with 19 ml of 93 % ethanol. The separating property was very good. The amount of N^{ω}-trifluoroacetyl-L-lysine obtained was 15.8 g (65 mmol) and the purity was 99 % (w/w) or higher (N^{α}-isomer was not detected and sodium chloride content was 0.1 % (w/w) or lower). 4.4 g (18 mmol) of N^{ω}-trifluoroacetyl-L-lysine was contained in the filtrate and washing solution. The reaction yield (formation rate) was 75 mol%.

### EXAMPLE 6

A reaction mixture (pH 11.0) having good liquid condition obtained in the same manner as in Example 3 was added dropwise into a flask containing 10 ml of water at 50° to 60°C. During the addition, a concentrated hydrochloric acid was added dropwise at the same time over 0.5 hour while mildly stirring (around 0.1 kW/m³) and the pH of the solution in the flask and the inner temperature were maintained at 3 to 6 and 50° to 60°C, respectively. Finally, a slight amount of a 9.5 N aqueous sodium hydroxide solution was added to adjust the pH to 5.5 and the mixture was cooled to an inner temperature of about 5°C. The crystals were centrifuged and washed with 19 ml of 93 % ethanol. The separating property was very good. The amount of N^{ω}-trifluoroacetyl-L-lysine obtained was 16.3 g (67 mmol) and the purity was 99 % (w/w) or higher (N^{α}-isomer was not detected and sodium chloride content was 0.1 % (w/w) or lower). The crystallization yield was 78 mol%. The total yield was 61 mol%.

### EXAMPLE 7

To 80 ml of water was added 20.0 g (110 mmol) of L-lysine monohydrochloride and the resultant was adjusted to pH 10.0 with a 9.5N aqueous sodium hydroxide solution. The mixture was cooled to an inner temperature of 3°C and 15.6 g (110 mmol) of ethyl trifluoroacetate was slowly added dropwise under stirring over 20 minutes. During the addition, the reaction mixture was maintained at pH 9.9 to 10.5 while slowly adding a 9 N aqueous sodium hydroxide solution and, at the same time, the inner temperature and the agitation power were maintained at 2° to 7°C and 0.5 to 1 kW/m³, respectively. 10 minutes after completion of the addition of ethyl trifluoroacetate, 22 g of a concentrated hydrochloric acid was added all at once to the whip-like reaction mixture to dissolve it. The pH after the addition was 1.0 or lower. The formation rate of N^{ω}-trifluoroacetyl-L-lysine was 63 mol%. The solution was warmed to 65°C, a 9.5N aqueous sodium hydroxide solution was added at an approximately constant rate at an agitation power of 0.2 kW/m³ over 20 minutes to adjust the pH to 5.5. The solution was cooled to an inner temperature of 5°C and maintained at that temperature overnight. The crystals were centrifuged and washed with 19 ml of 93 % ethanol. The separating property was very good. The amount of N^{ω}-trifluoroacetyl-L-lysine obtained was 13.1 g (54 mmol) and the purity was 99 % (w/w) or higher (N^{α}-isomer was not detected and sodium chloride content was 0.1 % (w/w) or lower. The crystallization yield was 77 mol%.

This Example indicates as follows: Since formation of N^{ω}-trifluoroacetyl-L-lysine was performed outside the range of the present invention (pH 10.6 to 11.4), the reaction mixture became whip-like. However, since the operations for crystallization and collection were performed according to the present invention, N^{ω}-trifluoroacetyl-L-lysine having good crystal property could be obtained at good operability.

### COMPARATIVE EXAMPLE 1

Into 350 ml of water and 125 ml (0.50 mol) of a 4N aqueous sodium hydroxide solution in a 1000 ml cylindrical flask (using a paddle type agitator (60 mm)) was dissolved 91.3 g (0.50 mol) of L-lysine monohydrochloride at 10° to 20°C. This solution (pH 10.4) was cooled to 4°C and 71.1 g (0.50 mol) of ethyl trifluoroacetate was rapidly added thereto under vigorous stirring (700 rpm). After the addition, vigorous stirring was continued for 2 hours while maintaining the inner temperature at around 5°C . During this process, a whip-like product was precipitated from the reaction mixture and the mixture became in a condition where mixing by stirring became impossible (after start of the reaction, the pH of the reaction mixture was gradually lowered to 8.7). Finally, the reaction mixture at pH 6 was filtered under reduced pressure using a nutsche having a diameter of 150 mm and the crystals were washed with 200 ml of water and 150 ml of 99.5 % ethanol in this order. Since the crystal property was bad, it took 1 hour or longer for filtration and washing. The resulting crystals were vacuum-dried at 50°C overnight for sufficient drying. The cake was shrank to give a dry lump. The resultant crystals had a purity of 93 % (w/w) and a water and ethanol contents of less than 1 % (w/w) and contained 47.3 g (0.20 mmol, yield 40 mol%) of N^{ω}-trifluoroacetyl-L-lysine as a net amount. In addition, 13.7 g (0.06 mol, yield 11 mol%) of N^{ω}-trifluoroacetyl-L-lysine was contained in the filtrate, 9.8 g (0.04 mol, yield 8 mol%) of N^{ω}-trifluoroacetyl-L-lysine was contained in the water washing solution and 4.7 g (0.02 mol, yield 4 mol%) of N^{ω}-trifluoroacetyl-L-lysine was contained in the ethanol washing solution. The reaction yield (formation rate) was 63 mol%.

### EXAMPLE 8 AND COMPARATIVE EXAMPLES 2 TO 4

To 37.5 ml of water was added 9.13 g (50.0 mmol) of L-lysine monohydrochloride and the resultant was adjusted to a predetermined pH value (pH 13.0, 12.0, 11.0 or 10.0) with a 4 N aqueous sodium hydroxide solution. After cooling to an inner temperature of 4°C, 7.11 g (50.0 mmol) of ethyl trifluoroacetate was slowly added dropwise thereto under stirring over 30 minutes. During the addition, the reaction mixture was maintained at the predetermined pH (pH 13.0, 12.0, 11.0 or 10.0) while adding a 4 N aqueous sodium hydroxide solution and, at the same time, the inner temperature and the agitation power were maintained at 4°C and 1.5 kW/m³, respectively. Fifteen minutes after completion of the addition of ethyl trifluoroacetate, a concentrated hydrochloric acid was added to adjust the reaction mixture to pH 1, and the mixture was quenched and subjected to HPLC analysis to determine the formation rate of N^{ω} -trifluoroacetyl-L-lysine and the ω / α selectivity (N^{ω} isomer (mmol)/N^{α} isomer (mmol)). The results are shown in Table 1.

**TABLE 1**

| Ex.No.and Com.Ex.No. | pH | Formation rate (mol%) | ω / α Selectivity |
|---|---|---|---|
| Com.Ex.2 | 13.0 | 16 | 88/12 |
| Com.Ex.3 | 12.0 | 64 | 99/1 |
| Ex.8 | 11.0 | 83 | 94/6 |
| Com.Ex.4 | 10.0 | 60 | 89/11 |

In addition, at pH 10.0, a whip-like product was precipitated during the reaction and it was observed that a slight amount of ethyl trifluoroacetate remained.

### COMPARATIVE EXAMPLE 5

To 47.5 ml of water was added 9.13 g (50.0 mmol) of L-lysine monohydrochloride, and 2.6 g of sodium carbonate and 4.2 g of sodium hydrogencarbonate were added thereto. After cooled to an inner temperature of 4°C, 7.11 g (50.0 mmol) of ethyl trifluoroacetate was slowly added dropwise under stirring over 30 minutes. During the addition, the reaction mixture was maintained at pH 9.0 while adding a 4 N aqueous sodium hydroxide solution dropwise and, at: the same time, the inner temperature and the agitation power were maintained at 4°C and 1.5 kW/m³, respectively. Fifteen minutes after completion of the addition of ethyl trifluoroacetate, a concentrated hydrochloric acid was added to adjust the reaction mixture to pH 1, and the mixture was quenched and subjected to HPLC analysis. It was found that the formation rate of N^{ω}-trifluoroacetyl-L-lysine was 29 mol% and the *ω* / α selectivity (N^{ω} isomer (mmol)/N^{α} isomer (mmol)) was 65/35.

### EXAMPLES 9 TO 11

To 37.5 ml of water was added 9.13 g (50.0 mmol) of L-lysine monohydrochloride and the resultant was adjusted to pH 11.0 with a 4N aqueous alkali metal hydroxide solution shown in Table 2 in each case. After cooled to an inner temperature of 4°C, 7.11 g (50.0 mmol) of ethyl trifluoroacetate was slowly added dropwise thereto under stirring over 30 minutes. During the addition, the reaction mixture was maintained at pH 11.0 while adding dropwise each 4N aqueous alkali metal hydroxide solution and, at the same time, the inner temperature and the agitation power were maintained at 4°C and 1.5 kW/m³, respectively. 15 minutes after completion of the addition of ethyl trifluoroacetate, a concentrated hydrochloric acid was added to adjust the pH of the reaction mixture to 1 and the resultant was subjected to HPLC analysis to determine the formation rate of N^{ω}-trifluoroacetyl-L-lysine and the *ω* / α selectivity (N^{ω} isomer (mmol)/N^{α} isomer (mmol)). The results are shown in Table 2.

**TABLE 2**

| Ex.No. | Kind of alkali metal hydroxide | Formation rate (mol%) | ω / α selectivity |
|---|---|---|---|
| 9 | Sodium hydroxide | 83 | 97/3 |
| 10 | Potassium hydroxide | 77 | 98/2 |
| 11 | Lithium hydroxide | 79 | 98/2 |

Precipitation of the product during the reaction was not observed in every case and each reaction mixture was in good liquid condition.

### EXAMPLE 12

To 50 ml of water was added 18.3 g (0.10 mol) of L-lysine monohydrochloride and the resultant was adjusted to pH 11.0 with a 9 N aqueous sodium hydroxide solution. After cooled to an inner temperature of 4°C, 14.2 g (0.10 mol) of ethyl trifluoroacetate was slowly added dropwise thereto under stirring over 30 minutes. During the addition, the reaction mixture was maintained at pH 11.0 while adding dropwise a 9 N aqueous sodium hydroxide solution and, at the same time, the inner temperature and the agitation power were maintained at 4°C and 1.5 kW/m³, respectively. 15 minutes after completion of the addition of ethyl trifluoroacetate, a concentrated hydrochloric acid was added to adjust the pH of the reaction mixture to 1 and the resultant was subjected to HPLC analysis. As a result, it was found that the formation rate of N^{ω}-trifluoroacetyl-L-lysine was 75 mol% and the *ω /* α selectivity (N^{ω} isomer (mmol)/ N^{α} isomer (mmol)) was 99/1.

### EXAMPLE 13 AND COMPARATIVE EXAMPLE 6

According to the same manner as in Example 2, a reaction mixture was obtained. After 340 g of the reaction mixture was added to a mixture (65°C ) of 70 ml of water and 22 g of a concentrated hydrochloric acid under mild stirring (0.2 kW/m³) over 30 minutes, the mixture was cooled to 10°C over 1 hour to obtain a slurry at pH 6.0 (Slurry A). Separately, after 70 ml of water and 22 g of a concentrated hydrochloric acid were added to 340 g of the reaction mixture under stirring (0.2 kW/m³) in a range of 5° to 25°C over 30 minutes, the mixture was cooled to 10°C over 1 hour to obtain a slurry at pH 6.0 (Slurry B).

Slurry A had extremely good fluidity, whereas Slurry B was whip-like and, even when turned the container upside down, the slurry did not drop down.

In addition, when the whole amount of each slurry was filtered using a nutsche having a diameter of 150 mm at a differential pressure of 0.97325 bar (730 mm Hg), the filtrate passing rate (expressed in terms of a time required for obtaining a predetermined amount of the filtrate) was measured as shown in Table 3. As regards Slurry B, after the slurry was released on a nutsche while scraping with a spatula, the filtration was initiated.

**TABLE 3**

| | Ex.13 (Slurry A) | Com.Ex.6 (Slurry B) |
|---|---|---|
| Amount of filtrate (ml) | Filtrate passing rate(s) | |
| 100 | 2 | 3 |
| 200 | 5 | 12 |
| 300 | 9 | 65 |

It was found that the filtration property of Slurry A was considerably better than that of Slurry B. In addition, the shrinkage of the cake of Slurry B was very large.

The resultant crystals were observed with a microscope. As a result, the crystals of Slurry A had a particle size of about 100 to 200 *µ*m or larger, whereas the crystals of Slurry B had a particle size of about 20 *µ*m or smaller.

According to the present invention, high quality N^{ω}-trifluoroacetyl-basic amino acid can be advantageously and economically prepared in a high yield on a commercial scale.

## Claims

1. A process for preparing N^{ω}-trifluoroacetyl-basic amino acid from a basic amino acid and a trifluoroacetic ester which comprises: adding successively a trifluoroacetic ester to a basic aqueous liquid containing a basic amino acid and a basic pH adjusting agent at a concentration at which a total weight of the basic amino acid is 5 to 40 % (w/v) relative to the volume of a charged solvent while maintaining the pH of the aqueous liquid in a range of 10.6 to 11.4 and the temperature at 20°C or lower, thereby performing the reaction, and crystallizing and collecting N^{ω}-trifluoroacetyl-basic amino acid from the aqueous liquid after the reaction.

2. The process of Claim 1, wherein the successive addition of the trifluoroacetic ester to the basic aqueous liquid is performed while stirring at an agitation power of not less than 1/10 kW/m³.

3. The process of Claim 1 or 2, wherein the successive addition of the trifluoroacetic ester is performed over a range of 1/4 to 8 hours.

4. The process of Claim 1, 2 or 3, wherein the amount of the trifluoroacetic ester to be added is from 0.5 to 2 by mole ratio relative to the basic amino acid.

5. A process for crystallizing and collecting N^{ω}-trifluoroacetyl-basic amino acid from an aqueous liquid in which N^{ω}-trifluoroacetyl-basic amino acid is dissolved which comprises growing the crystals in said aqueous liquid while adjusting the pH to a range of the isoelectric point of N^{ω}-trifluoroacetyl-basic amino acid ±3 under neutral or acidic conditions at a temperature of 40°C or higher, wherein the aqueous liquid in which N^{ω}-trifluoroacetyl-basic amino acid is dissolved is a reaction mixture obtained by successively adding a trifluoroacetic ester to a basic aqueous liquid containing a basic amino acid and a basic pH adjusting agent while maintaining the pH of said basic aqueous liquid in a range of 10.6 to 11.4 and the temperature at 20°C or lower, thereby performing the reaction.

6. The process of Claim 5, wherein the growth of N^{ω}-trifluoroacetyl-basic amino acid crystals is performed while stirring at an agitation power of 0.01 to 0.5 kW/m³.

7. The process of Claim 5 or 6, wherein the pH is finally adjusted to a range of 5 to 6 in the growth of N^{ω}-trifluoroacetyl-basic amino acid crystals.

8. The process of Claim 5, 6 or 7, wherein after the growth of N^{ω} -trifluoroacetyl-basic amino acid crystals, crystallization is further performed by cooling to 20°C or lower.

9. The process of Claim 5, 6, 7 or 8, wherein upon collection of N^{ω}-trifluoroacetyl-basic amino acid crystals, the crystals are separated from the slurry, and subsequently the filtrate contained in the wet crystals is washed and removed using an alcohol having 1 to 3 carbon atoms.

10. The process of any of Claims 5-9, wherein the successive addition of the trifluoroacetic ester to said basic aqueous liquid is performed over a range of 1/4 to 8 hours.

11. The process of any of Claims 5-10, wherein the successive addition of the trifluoroacetic ester to said basic aqueous solution is performed while stirring at an agitation power of not less than 1/10 kW/m³.

12. The process of any of Claims 5-11, wherein the amount of the trifluoroacetic ester to be added is from 0.5 to 2 by mole ratio relative to the basic amino acid.

13. The process of any one of Claims 5 to 12, wherein the basic amino acid is lysine.

## Patentansprüche

1. Verfahren zur Herstellung von N^{ω}-trifluoracetylierter basischer Aminosäure aus einer basischen Aminosäure und einem Trifluoressigsäureester, welches umfasst: sukzessive Zugabe eines Trifluoressigsäureesters zu einer basischen wässerigen Flüssigkeit, die eine basische Aminosäure und ein einen basischen pH regulierendes Mittel in einer Konzentration enthält, bei welcher das Gesamtgewicht der basischen Aminosäure 5 bis 40 % (w/v) bezogen auf das Volumen eines vorgelegten Lösungsmittels beträgt, wobei der pH-Wert der wässerigen Flüssigkeit in einem Bereich von 10,6 bis 11,4 und die Temperatur bei 20°C oder darunter gehalten wird, wodurch die Reaktion ausgeführt wird, sowie Kristallisieren und Sammeln der N^{ω} -trifluoracetylierten basischen Aminosäure aus der wässerigen Flüssigkeit nach der Umsetzung.

2. Verfahren nach Anspruch 1, wobei die sukzessive Zugabe des Trifluoressigsäureesters zu der basischen wässerigen Flüssigkeit unter Rühren mit einer Rührleistung von mindestens 1/10 kW/m³ vorgenommen wird.

3. Verfahren nach Anspruch 1 oder 2, wobei die sukzessive Zugabe des Trifluoressigsäureesters in einer Zeitspanne von 1/4 bis 8 Stunden vorgenommen wird.

4. Verfahren nach Anspruch 1, 2 oder 3, wobei die zuzusetzende Menge des Trifluoressigsäureesters ein molares Verhältnis von 0,5 bis 2, bezogen auf die basische Aminosäure, ausmacht.

5. Verfahren zum Kristallisieren und Sammeln von N^{ω}-trifluoracetylierter basischer Aminosäure aus einer wässerigen Flüssigkeit, in der die N^{ω}-trifluoracetylierte basische Aminosäure gelöst ist, umfassend das Wachsen der Kristalle in der wässerigen Flüssigkeit unter gleichzeitigem Einstellen des pH-Wertes auf einen Bereich entsprechend dem isoelektrischen Punkt der N^{ω}-trifluoracetylierten basischen Aminosäure ± 3 unter neutralen oder sauren Bedingungen bei einer Temperatur von 40°C oder höher, wobei die wässerige Flüssigkeit, in der die N^{ω} -trifluoracetylierte basische Aminosäure gelöst ist, ein Reaktionsgemisch ist, das durch sukzessive Zugabe eines Trifluoressigsäureesters zu einer basischen wässerigen Flüssigkeit erhalten wird, welche eine basische Aminosäure und ein einen basischen pH regulierendes Mittel enthält, wobei der pH-Wert der basischen wässerigen Flüssigkeit in einem Bereich von 10,6 bis 11,4 und die Temperatur bei 20°C oder darunter gehalten wird, wodurch die Reaktion ausgeführt wird.

6. Verfahren nach Anspruch 5, wobei das Wachsen der Kristalle der N^{ω}-trifluoracetylierten basischen Aminosäure unter Rühren mit einer Rührleistung von 0,01 bis 0,5 kW/m³ durchgeführt wird.

7. Verfahren nach Anspruch 5 oder 6, wobei der pH-Wert beim Wachsen der Kristalle der N^{ω}-trifluoracetylierten basischen Aminosäure abschließend auf einen Bereich von 5 bis 6 eingestellt wird.

8. Verfahren nach Anspruch 5, 6 oder 7, wobei nach dem Wachsen der Kristalle der N^{ω}-trifluoracetylierten basischen Aminosäure die Kristallisation durch Abkühlen auf 20°C oder darunter weiter ausgeführt wird.

9. Verfahren nach Anspruch 5, 6, 7 oder 8, wobei beim Sammeln der Kristalle der N^{ω}-trifluoracetylierten basischen Aminosäure die Kristalle von der Trübe getrennt werden und danach das in den nassen Kristallen enthaltene Filtrat unter Verwendung eines Alkohols mit 1 bis 3 Kohlenstoffatomen ausgewaschen und entfernt wird.

10. Verfahren nach einem der Ansprüche 5 - 9, wobei die sukzessive Zugabe des Trifluoressigsäureesters zu der basischen wässerigen Flüssigkeit über eine Zeitspanne von 1/4 bis 8 Stunden vorgenommen wird.

11. Verfahren nach einem der Ansprüche 5 - 10, wobei die sukzessive Zugabe des Trifluoressigsäureesters zu der basischen wässerigen Lösung unter Rühren bei einer Rührleistung von mindestens 1/10 kW/m³ vorgenommen wird.

12. Verfahren nach einem der Ansprüche 5 - 11, wobei die zuzusetzende Menge des Trifluoressigsäureesters ein molares Verhältnis von 0,5 bis 2, bezogen auf die basische Aminosäure, ausmacht.

13. Verfahren nach einem der Ansprüche 5 bis 12, wobei die basische Aminosäure Lysin ist.

## Revendications

1. Procédé pour préparer un acide N^{ω}-trifluoroacétylaminé basique à partir d'un acide aminé basique et d'un ester trifluoroacétique qui comprend : l'addition successive d'un ester trifluoroacétique à un liquide aqueux basique contenant un acide aminé basique et un agent d'ajustement de pH basique à une concentration à laquelle la masse totale de l'acide aminé basique est 5 à 40 % (m/v) par rapport au volume d'un solvant introduit tout en maintenant le pH du liquide aqueux dans un domaine de 10,6 à 11,4 et la température à 20°C ou inférieure, pour conduire la réaction, et la cristallisation et la récupération de l'acide N^{ω}-trifluoroacétylaminé basique à partir du liquide aqueux après la réaction.

2. Procédé selon la revendication 1, où l'addition successive de l'ester trifluoroacétique au liquide aqueux basique est réalisée tout en agitant à une puissance d'agitation d'au moins 1/10 kW/m³.

3. Procédé selon la revendication 1 ou 2, où l'addition successive de l'ester trifluoroacétique est conduite dans un domaine de 1/4 à 8 h.

4. Procédé selon la revendication 1, 2 ou 3, où. la quantité d'ester trifluoroacétique à ajouter est de 0,5 à 2 en rapport molaire par rapport à l'acide aminé basique.

5. Procédé pour cristalliser et récupérer un acide N^{ω}-trifluoroacétylaminé basique à partir d'un liquide aqueux dans lequel un acide N^{ω}-trifluoroacétylaminé basique est dissous qui comprend la croissance des cristaux dans ledit liquide aqueux tout en ajustant le pH dans un domaine du point isoélectrique de l'acide N^{ω}-trifluoroacétylaminé basique ± 3 dans des conditions neutres ou acides à une température de 40°C ou supérieure, où le liquide aqueux dans lequel l'acide N^{ω}-trifluoroacétylaminé basique est dissous est un mélange réactionnel obtenu par addition successive d'un ester trifluoroacétique à un liquide aqueux basique contenant un acide aminé basique et un agent d'ajustement de pH basique tout en maintenant le pH dudit liquide aqueux basique dans un domaine de 10,6 à 11,4 et la température à 20°C ou inférieure, pour conduire la réaction.

6. Procédé selon la revendication 5, où la croissance de cristaux d'acide N^{ω}-trifluoroacétylaminé basique est conduite tout en agitant à une puissance d'agitation de 0,01 à 0,5 kW/m³.

7. Procédé selon la revendication 5 ou 6, où le pH est finalement ajusté dans un domaine de 5 à 6 dans la croissance de cristaux d'acide N^{ω}-trifluoroacétylaminé basique.

8. Procédé selon la revendication 5, 6 ou 7, où, après la croissance de cristaux d'acide N^{ω}-trifluoroacétylaminé basique, la cristallisation est conduite encore par refroidissement à 20°C ou à une température plus basse.

9. Procédé selon la revendication 5, 6, 7 ou 8, où, lors de la récupération de cristaux d'acide N^{ω}-trifluoroacétylaminé basique, les cristaux sont séparés de la suspension, puis le filtrat contenu dans les cristaux humides est lavé et retiré au moyen d'un alcool ayant 1 à 3 atomes de carbone.

10. Procédé selon l'une quelconque des revendications 5 à 9, où l'addition successive de l'ester trifluoroacétique audit liquide aqueux basique est accomplie dans un domaine de 1/4 à 8 h.

11. Procédé selon l'une quelconque des revendications 5 à 10, où l'addition successive de l'ester trifluoroacétique à ladite solution aqueuse basique est accomplie tout en agitant à une puissance d'agitation d'au moins 1/10 kW/m³.

12. Procédé selon l'une quelconque des revendications 5 à 11, où la quantité d'ester trifluoroacétique à ajouter est de 0,5 à 2 en rapport molaire par rapport à l'acide aminé basique.

13. Procédé selon l'une quelconque des revendications 5 à 12, où l'acide aminé basique est la lysine.
